# EUROPEAN PATENT SPECIFICATION

(11) **EP 1 759 645 B1**
(45) Date of publication and mention of the grant of the patent: **05.11.2008**
(21) Application number: 06018482.7
(22) Date of filing: 04.09.2006
(51) Int. Cl.: A61B 17/34, A61M 29/00

(54) **Instrument introducer**
Einführeinrichtung für ein Instrument
Module d'introduction d'instrument

(30) Priority: 06.09.2005 US 220263
(43) Date of publication of application: 07.03.2007
(73) Proprietor: Tyco Healthcare Group LP, Norwalk, Connecticut 06856 (US)
(72) Inventor: Milliman, Keith L., Bethel, CT 06801 (US)
(74) Representative: HOFFMANN EITLE

(56) References cited:
- WO-A-84/01512
- WO-A-02/051323
- WO-A2-20/05009257

## Description

### BACKGROUND

### 1. Technical Field

The present disclosure relates to instrument introducers or protective sleeves and methods of using the same and, more particularly, to novel protective sheaths configured to facilitate the introduction of surgical instruments into a cavity, an orifice or a body opening of a patient.

### 2. Background of Related Art

Surgical instruments configured for remote use inside the body of a patient typically define a central longitudinal axis including a distal end portion and a proximal end portion. The distal end portion of the surgical instrument can include configurations which have either blunt or rounded faces and/or in certain instances include sharper subassemblies including electrosurgical and/or mechanical blades for cutting as well as fasteners for securing tissue portions. As a result of the mechanical complexity of these surgical instruments, the distal end portions of these instruments have been provided with a shell or cover that may partially or fully enclose the distal end portion of the surgical instrument.

For example, presently, various intra-anal surgical instruments, such as intraluminal anastomotic surgical staplers, require insertion into the colon or intestine through the anus. In certain embodiments, these surgical staplers have staple anvil portions removably mounted to a distal end thereof. Generally, the surgical stapler is inserted with the anvil portion attached, however, certain surgical procedures require that the surgical stapler be inserted into the colon or intestine through the anus with the anvil portion removed therefrom.

Typically, in instances where the surgical stapler is to be inserted into the colon or intestine of the patient with the anvil portion mounted to a distal end thereof, the anvil portion is tapered toward the tip and formed to have an atraumatic end, thus facilitating the insertion of the distal end of the surgical instrument. However, in such instances or in instances where the surgical stapler is to be inserted into the colon or intestine without the anvil portion mounted to a distal end thereof, it is desirable to have an instrument and/or accessory which reduces potential irritation and/or trauma to the surrounding tissue which may result from, for example, the substantially squared or non-tapered distal end of the surgical instrument.

Thus, a need exists for a instrument introducer in the form of a sheath, which instrument introducer facilitates the passage of the surgical instrument into the body of the patient and that can be either adapted to be removably mounted on a distal end of the surgical instrument or be used as a separate device adapted to be positioned at least partially into the body of the patient (i.e., into the anus) and wherein the surgical instrument is subsequently positioned into the instrument introducer to facilitate insertion of the surgical instrument into the body of the patient.

WO 021051323 discloses an instrument according to the preamble of claim 1 having an adaptation element on the proximal end and a sleave attached thereto and comprising a fold along its wall.

WO 84/01512 discloses a tubular guiding device made of a thin and flexible material to allow its cross-section to be reduced.

### SUMMARY

The present invention comprises instrument introducers as defined in claim 1 with preferred embodiments as disclosed in the dependent claims. The instrument introducers being configured to facilitate the introduction of surgical instrument into a body opening of a patient. In one aspect of the present disclosure, the instrument introducer includes a body portion having a distal end portion including a distal orifice, and a proximal end portion including a proximal orifice. The body portion defines a lumen for passage of a portion of a surgical instrument therethrough. The distal end portion includes at least one fold formed therein. The distal end portion can have a plurality of folds extending radially thereabout. The plurality of folds can be configured and dimensioned to define a distal end portion having a tapered configuration, for example, a generally conical or frusto-conical shape.

At least the distal end portion of the instrument introducer can be fabricated from at least one or a combination of suitable materials, for example, a polypropylene, a moldable plastic, a thermoformable plastic, a polymer, a urethane, a silicone, a natural rubber, a synthetic rubber, an elastomer, an elastomeric material, and a latex material. The instrument introducer can be fabricated from a material having a durometer reading of about 5A to about 90A, preferably, from about 20A to about 70A. Preferably, the material of at least the distal end portion is flexible, e.g., elastic, stretchable or yieldable to allow the folds to partly or fully unfold.

According to another aspect of the present disclosure, the instrument introducer includes a body portion defining a lumen for passing a portion of the surgical instrument therethrough. The body portion includes a distal end portion fabricated from a flexible material and including a distal orifice, a proximal end portion including a proximal orifice, and at least one fold formed in at least the distal end portion of the body portion.

The at least one fold provides the distal end portion with a taper extending in a distal direction. The distal end portion of the instrument introducer has a first condition having a generally conical shape. In the first condition, the distal end portion of the instrument introducer has a generally frusto-conical shape. Additionally, the distal end portion of the instrument introducer may be comprised of a plurality of folds extending radially about the distal end portion. Desirably, the folds extend in a longitudinal direction. The folds provide the distal end portion with a taper extending in a distal direction.

The distal end portion of the instrument introducer has a first condition wherein the folds are unexpanded to facilitate insertion of the distal end portion into a body orifice. The distal end portion of the instrument introducer has one or more subsequent conditions wherein the folds are at least partially extended to accommodate the passage of a surgical instrument therethrough. The distal end portion of the instrument introducer has a subsequent condition having an extended internal diameter substantially equal to an outer diameter of a surgical instrument.

Desirably, the folds are integral with one another. It is envisioned that the folds may overlap one another. In one embodiment, the body portion of the instrument introducer has an inner diameter of from about 30 mm (1.2 inches) to about 40 mm (1.6 inches).

The instrument introducer may be fabricated from at least one of a polypropylene, a moldable plastic, a thermoformable plastic, a polymer, a urethane, a silicone, a natural rubber, a synthetic rubber, an elastomer, an elastomeric material, and a latex material.

It is envisioned that the instrument introducer includes at least one fold which extends along the entire length of the body portion. The folds may be in the form of pleats. The body portion of the instrument introducer may include at least one annular fold formed about the body portion.

According to another aspect of the present disclosure, an exemplary method for inserting a distal end portion of a surgical instrument into a body opening of a patient, is disclosed. The method includes the step of providing an instrument introducer including a body portion defining a lumen therethrough for accommodating a distal end portion of a surgical instrument therethrough. The body portion may include a distal end portion having a distal orifice, a proximal end portion defining a proximal orifice, and at least one fold formed in at least the distal end portion of the body portion, wherein at least the distal end portion of the instrument introducer is fabricated from a flexible material.

The method further includes the steps of positioning the distal end portion of the instrument introducer into the body opening of a patient, inserting the distal end portion of the surgical instrument into the instrument introducer, and advancing the distal end portion of the surgical instrument through the lumen of the instrument introducer, such that as the distal end portion of the surgical instrument is passed into the distal end portion of the instrument introducer, the at least one fold radially expands to accommodate the distal end portion of the surgical instrument.

It is envisioned that the instrument introducer may include a plurality of folds extending radially around the distal end portion, and the plurality of folds may expand to accommodate the distal end portion of the surgical instrument. The folds may extend in a longitudinal direction such that during the advancing stage the folds gradually expand as the surgical instrument is passed into the distal end portion of the instrument introducer.

The distal end portion of the instrument introducer may have an initial condition wherein the folds are unexpanded and during the advancing step, the folds expand to facilitate insertion of the distal end portion of the instrument introducer into the body opening. Desirably, during the advancing step, the distal end portion of the instrument introducer may have a plurality of subsequent conditions wherein the folds are at least partially extended to accommodate movement of the distal end portion of the surgical instrument therethrough. Additionally, during the advancing step, the distal end portion of the instrument introducer may have an extended condition having an internal diameter substantially equal to an outer diameter of the distal end portion of the surgical instrument passing therethrough.

The method further includes the step of advancing the distal end portion of the surgical instrument through the distal orifice of the instrument introducer.

Desirably, at least the distal end portion of the instrument introducer is fabricated from at least one flexible material, and during the advancing step, the flexible material of the folds extend.

The method may further include the step of providing a lubricant on at least one of an inner surface of the instrument introducer and an outer surface of the distal end portion of the surgical instrument.

The presently disclosed instrument introducer, together with attendant advantages, will be more clearly disclosed below in the drawings and in the description of the drawings and of the preferred embodiments.

### BRIEF DESCRIPTION OF THE DRAWINGS

By way of example only, embodiments of the disclosure will be described with reference to the accompanying drawings, in which:
FIG. 1 is a top perspective view of an instrument introducer in accordance with an embodiment of the present disclosure;
FIG. 2 is a top plan view of the instrument introducer of FIG. 1;
FIG. 3 is a bottom plan view of the instrument introducer of FIGS. 1 and 2;
FIG. 4 is a side elevational view of the instrument introducer of FIGS. 1-3;
FIG. 5 is a cross-sectional side elevational view of the instrument introducer of FIGS. 1-4, as taken through a longitudinal axis thereof;
FIG. 6 is a perspective view of an instrument introducer in accordance with another embodiment of the present disclosure;
FIG. 7 is a side elevational view of the instrument introducer of FIG. 6;
FIG. 8 is a top plan view of the instrument introducer of FIGS. 6 and 7 prior to radial expansion thereof;
FIG. 9 is a top plan view of the instrument introducer of FIGS. 6-8 shown in a radially expanded condition with a surgical instrument extending therethrough;
FIG. 10 is a perspective view of an instrument introducer in accordance with another embodiment of the present disclosure;
FIG. 11 is a side elevational view of the instrument introducer of FIG. 10;
FIG. 12 is a side elevational view of the instrument introducer of FIGS. 10 and 11 whose body is in an extended and/or stretched state;
FIG. 13 is a side elevational view of the instrument introducer of FIGS. 10-12 in a bent and/or deflected state;
FIG. 14 is a top plan view of an instrument introducer in accordance with yet another embodiment of the present disclosure;
FIG. 15 is a top plan view of an instrument introducer in accordance with still another embodiment of the present disclosure;
FIG. 16 is a cross-sectional side elevational view, taken along the longitudinal axis, of the instrument introducer of FIGS. 1-5, depicting the initial insertion of a surgical instrument into the instrument introducer; and
FIG. 17 is a cross-sectional side elevational view, taken along the longitudinal axis, of the instrument introducer of FIGS. 1-5, depicting the complete insertion of a surgical instrument through the instrument introducer.

### DETAILED DESCRIPTION OF EMBODIMENTS

While the device according to the present disclosure is especially suitable for introducing a distal end portion of a surgical instrument into a body opening of a patient (for example, the anal orifice, the external urethral opening, the mouth or a natural or formed opening in a body cavity of a patient), for performing certain surgical procedures, (e.g., hemorrhoidal, mucosal prolapse or end-to-end anastomotic applications), it is envisioned that the device according to the present disclosure can be used in connection with other surgical instruments for performing any number of other endoscopic or laparoscopic surgical procedures. For example, it is believed that the device disclosed herein may find use in other procedures in which substantially blunt ended surgical instruments are introduced into body openings or cavities of patients.

In the drawings and in the description which follows, the term "proximal", as is traditional, will refer to the end of the surgical device or instrument of the present disclosure which is closest to the operator, while the term "distal" will refer to the end of the device or instrument which is furthest from the operator.

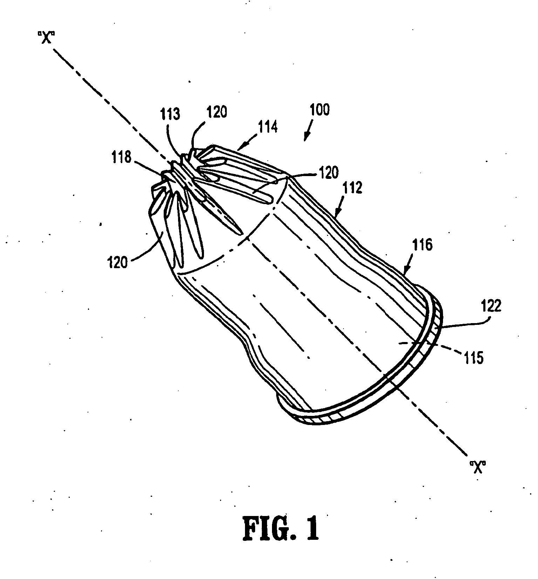

Referring now in specific detail to the drawings, in which like reference numerals identify similar or identical elements, FIGS. 1-4 illustrate a surgical instrument introducer generally designated as 100. Surgical instrument introducer 100 generally is in the form of a sheath, drape, sock, sleeve covering, casing, condom, etc. Accordingly, as used herein, instrument introducer 100 can embody any one of these terms.

As will be further described below, instrument introducer 100 is configured and adapted to be either removably mounted and/or placed on and/or over a distal end portion of a surgical instrument (e.g., anastomotic circular fastener applier or stapler) prior to insertion of the surgical instrument into an opening in the body, or is preferably configured and adapted for initial placement into the orifice of the body and for subsequent insertion of the distal end portion of the surgical instrument into and through the lumen of instrument introducer 100. Preferably, instrument introducer 100 has a shape which facilitates its entry into the orifice of the body and which is also compatible with the distal end portion of the surgical instrument. It is contemplated that instrument introducer 100 functions as a protective sheath or sleeve to cap, encase and/or surround the distal end portion of and to facilitate the entry of the distal end portion of the surgical instrument into the orifice of the body. In addition, instrument introducer 100 can act as an insulative barrier and/or an isolating barrier between the surgical instrument and the body of the patient.

As seen in FIGS. 1-5, instrument introducer 100 includes a hollow body portion 112 having a distal orifice 113, a distal end portion 114, a proximal orifice 115, and a proximal end portion 116. Body portion 112 defines a lumen 118 therethrough, which lumen 118 defines a central longitudinal axis "X". Preferably, body portion 112 has an inner diameter sized to receive a distal end portion of a surgical instrument therein. For example, for a circular stapler, body portion 112 can have an inner diameter of about about 30 mm to about 40 mm (1.2 to about 1.6 inches), preferably about 36 mm (1.4 inches).

It is envisioned that body portion 112 can vary in length depending on the type of surgical instrument that is to be inserted into the orifice of the body through lumen 118 and depending on the particular surgical application in which instrument introducer 100 is going to be used. Preferably, for example, for a circular stapler, instrument introducer 100 will vary in overall length from about 51 mm (2.0 inches) to about 61 mm (2.4 inches), preferably about 58 mm (2.2 inches).

Distal end portion 114 of instrument introducer 100 includes at least one fold, preferably a plurality of folds 120 formed or extending radially thereabout such that distal end portion 114 tapers linearly or arcuately or a combination thereof, in a distal direction to a radially smaller diameter at or adjacent distal orifice 113. Preferably, the at least one fold or a plurality of folds extend in a longitudinal direction. The fold(s) can extend along the entire length of body portion 112. As used herein, the term "fold" is understood to include pleat, undulation, corrugation, crease, bend and the like.
Generally, the fold(s) will be at or near distal orifice 113 such that the material that is folded, bent or undulating, etc., can open-up, unfold, extend or expand to allow the distal end of the instrument to pass through the orifice. Preferably, distal end portion 114 includes a plurality, e.g., an annular array, of folds 120 which in a first, e.g., at rest condition define a generally conical shape, more preferably, as shown, a generally or substantially frusto-conical configuration. While twelve folds 120 are shown, such should not be considered as limiting, as it is envisioned and within the scope of the present disclosure to have a distal end portion 114 including any number of folds greater or less than twelve. As shown in the Figures, the distal ends of the folds preferably are radially longer than their proximal ends. As will be described in greater detail below, the tapered configuration of distal end portion 114 facilitates insertion of instrument introducer 100 into the orifice of the patient.

Preferably, folds 120 are joined to or integral with one another and thus define a monolithic distal end portion 114. Alternatively, it is envisioned that folds 120 can be molded or formed to allow portions of folds 120 to extend over, overlie, overlap or rest on one another.

Distal end portion 114 has a first or initial configuration or condition, e.g., as formed or at rest, when no surgical instrument is extending therethrough, in which the fold or folds is or are unexpanded to facilitate insertion of the distal end portion into a body opening. In the initial condition, the portion of lumen 118 that extend through distal end portion 114 has a diameter which is less than the diameter of lumen 118 extending through body portion 112. Distal end portion 114 can have one or more subsequent or second configurations to accommodate the passage or extension of a surgical instrument therethrough, in which distal end portion 114 and/or distal orifice 113 is at least partially or fully extended or expanded to have a diameter which is substantially equal to the diameter of lumen extending through body portion 112. More particularly, in such configurations, distal orifice 113 or the portion of lumen 118 extending through body portion 112 has a diameter which is substantially equal to the outer diameter of the surgical instrument that is extending therethrough.

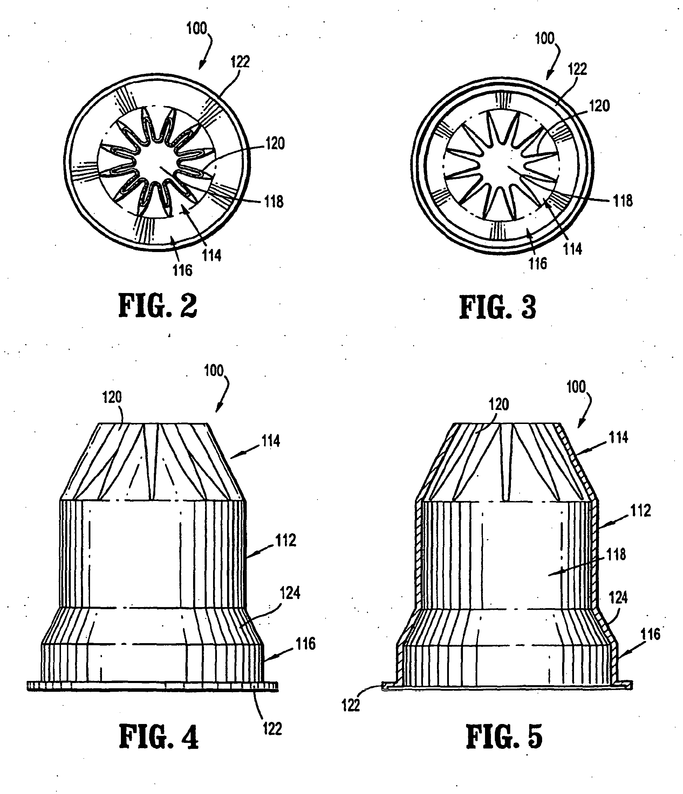

As seen in FIGS. 4 and 5, proximal end portion 116 preferably has a diameter which is larger than the radius of body portion 112 thereby defining a goose-neck portion 124 interconnecting body portion 112 with proximal portion 116. Proximal end portion has a size that facilitates insertion and entry of the surgical instrument into and through instrument introducer 100. For example, proximal end portion 116 can have an inner diameter of about 140 mm (5.4 inches) to about 240 mm (9.4 inches), preferably about 190 mm (7.4 inches).

The proximal end portion 116 of instrument introducer 100 preferably includes one or more radially outwardly extending portions such as one or more tabs or, more preferably, a radially outwardly extending continuous flange 122 integrally or monolithically formed with and/or connected to proximal end portion 116.

Flange 122 can be of any suitable length or width. For example, it can have one or more outwardly extending tabs, to provide one or more surfaces suitable for the placement of adhesives or tapes, e.g., such as those sold under the trademark Steri-strip, or of sutures to fix or secure instrument introducer 100 and potentially the surgical instrument relative to the patient and to limit the depth of penetration of instrument introducer 100 and/or the distal end of the surgical instrument into the body of the patient. While it is envisioned that flange 122 is to be used when instrument introducer 100 is employed as a stand alone device that is unconnected to a surgical instrument, it is envisioned that an instrument introducer 100 having a flange 122 can also be employed when instrument introducer 100 is connected to the surgical instrument prior to insertion of instrument introducer 100 into the cavity or body opening of the patient.

Instrument introducer 100 can be of any suitable wall thickness. For example, it can have a wall thickness of about 0.76 mm (0.03 inches) to about 3.8 mm (0.15 inches). Preferably, instrument introducer 100 has a uniform wall thickness throughout, however, the wall thickness can vary in portions or throughout.

Instrument introducer 100 can be fabricated from any suitable material or combinations of materials, for example, one or more moldable and/or thermoformable plastics, polymers, urethanes, natural or synthetic rubbers, silicones, elastomer and/or elastomeric or latex materials which is or are sufficiently extendible, expandible, pliable, malleable, ductile, compressible, elastic and/or rubbery to provide for controlled deflection and sufficient stiffness. Instrument introducer 100 is to be fabricated from an acceptable sterilizable medical grade material or combination of materials.

Instrument introducer 100 is preferably fabricated from a material having a reading on a Shore "A" durometer of generally about 20A to about 70A, but it could have a reading as low as 5A (the Shore "A" durometer reading for a hydrophillic contact lens) or about 90A (the Shore "A" durometer reading for a flexible acrylic material). Instrument introducer 100 can also be fabricated from a material having a reading on a Shore "D" durometer of about 40D to about 80D.

Other hardness scales can also be used. In general, durometers and their methods of use are described in ASTM D-2240. The Shore "A" scale can be used, for example, to measure the flexibility or relative hardness of synthetic rubbers, neoprene, silicones, felt, and the like. The Shore "B" scale can be used to measure the flexibility or alternatively the relative hardness of a variety of rubbers and elastomers. The Shore "C" scale is used to measure medium hard rubbers and plastics. The Shore "D" scale is used to measure a variety of plastics, plexiglass, polystyrene, vinyls, and the like. The Shore "OO" scale can be used to measure the hardness of materials such as sponges, rubber or soft rubber. Durometers are available from Pacific Transducer Corp., Los Angeles, Calif.

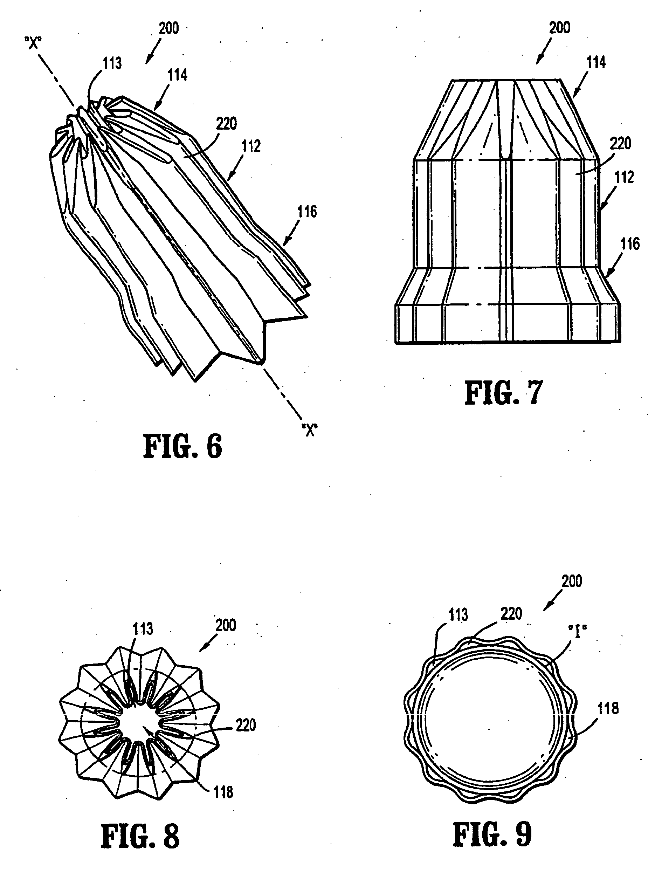

Turning now to FIGS. 6-9, an instrument introducer, in accordance with an alternative embodiment of the present disclosure, is generally designated 200. Instrument introducer 200 is similar to instrument introducer 100 and will only be described in detail to the extent necessary to identify differences in configuration, construction and/or operation.

As seen in FIGS. 6 and 7, instrument introducer 200 includes at least one, preferably a series of longitudinally oriented fold(s) 220, the series extending radially therearound. In an embodiment, folds 220 extend the entire length of instrument introducer 200 (i.e., from distal orifice 113, along distal end portion 114 and body portion 112, to proximal end portion 116). As such, the overall outer diameter of instrument introducer 200, and in this embodiment particularly of body portion 112, can be varied. In this manner, instrument introducer 200 can be inserted into orifices having relatively smaller diameters. In addition, as seen in FIG. 9, as a surgical instrument "I" is passed through distal orifice 113 and lumen 118 of instrument introducer 200, surgical instrument "I", if having a diameter which is larger than the diameter of orifice 113 and/or the diameter of lumen 118 of instrument introducer 200, causes lumen 118 and in turn orifice 113, to radially extend or expand.

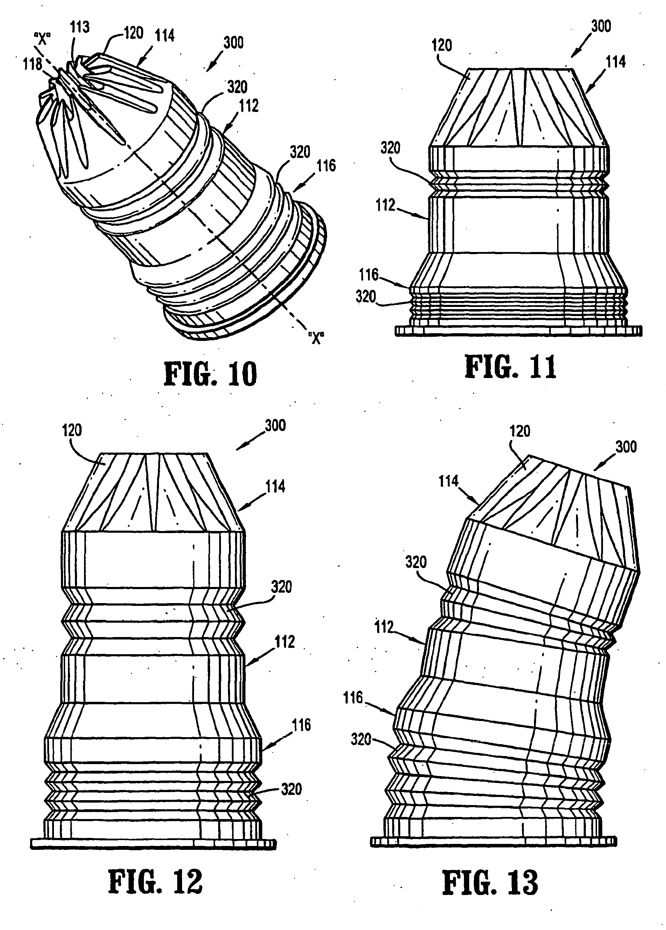

FIGS. 10-13 show another embodiment of an instrument introducer of the present disclosure, generally designated 300. Instrument introducer 300 is similar to instrument introducer 100 and will only be described in detail to the extent necessary to identify differences in configuration, construction and/or operation.

As seen in FIGS. 10-13, instrument introducer 300 includes at least one, preferably a plurality of, annular undulations or fold(s) 320, in the manner of an accordion, formed in body portion 112 and/or proximal end portion 116. In this manner, as seen in FIG. 12, as a surgical instrument is advanced through lumen 118 of instrument introducer 300, the friction between the inner surface of instrument introducer 300 and the outer surface of the surgical instrument, in addition to the forces required to advance the surgical instrument through and to radially expand distal end portion 114, causes instrument introducer 300 to extend and/or stretch distally, in a direction further into the body orifice. In addition, as seen in FIG. 13, annular fold(s) 320, formed in body portion 112 and/or proximal end portion 116, increase the overall flexibility of instrument introducer 300. In particular, folds 320 enable instrument introducer 300 to better conform (e.g., bend) to the shape or path of the body orifice and/or to better conform to the shape of the surgical instrument extending through lumen 118 thereof.

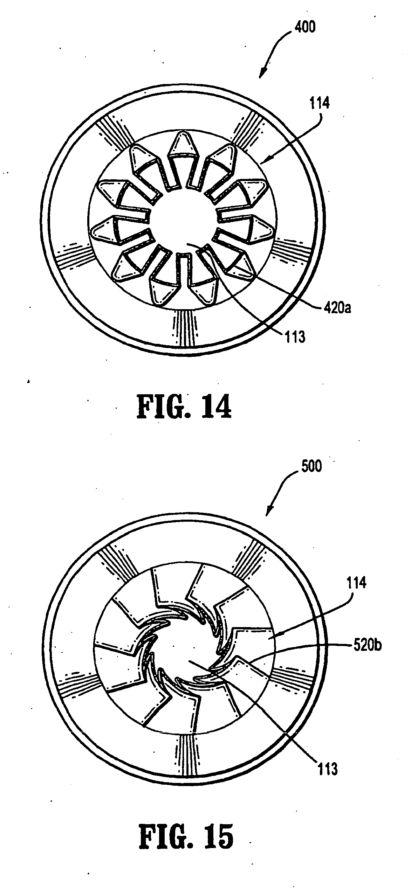

FIG. 14 shows another embodiment of an instrument introducer, generally designated 400. Instrument introducer 400 is similar to instrument introducer 100 and will only be described in detail to the extent necessary to identify differences in configuration, construction and/or operation.

As seen in FIG. 14, distal end portion 114 of instrument introducer 400 includes a plurality of pleats 420a, each having a substantially trapezoidal and/or triangular transverse cross-sectional profile, formed thereabout such that distal end portion 114 tapers in a distal direction to a radially smaller diameter.

FIG. 15 shows another embodiment of an instrument introducer, generally designated 500, having a distal end portion 114 that includes a plurality of pleats 520b, each having a folded-over and/or over-lapping configuration, formed therearound such that distal end portion 114 tapers in a distal direction to a radially smaller diameter.

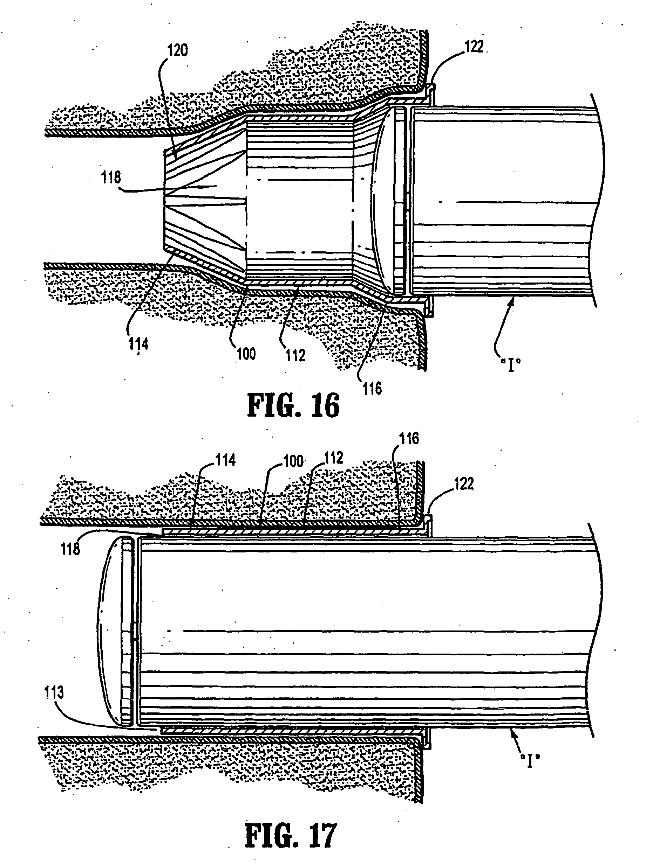

An exemplary use of instrument introducers 100-500 will now be described with reference to FIGS. 16 and 17. It is to be understood that use of each of instrument introducers 100-500 is substantially identical to one another and therefore, for the sake of clarity, simplicity and brevity, the following description will be directed to the use of instrument introducer 100.

In an embodiment of the method of use of an instrument introducer disclosed herein, instrument introducer, e.g., 100 is initially placed or positioned within a cavity or body opening or orifice of a patient (e.g., the anus) such that distal end portion 114 of instrument introducer 100 is retained therein at the desired depth. With at least distal end portion 114 of instrument introducer 100 in position, the distal end of a surgical instrument "I" is inserted into lumen 118 of instrument introducer 100 and advanced until the distal end of surgical instrument "I" contacts the inner surface of folds 120. As surgical instrument "I" is further advanced distally through instrument introducer 100, as seen in FIG. 17, the distal end portion of surgical instrument "I" begins to radially expand and/or stretch distal end portion 114 of instrument introducer 100, by unfolding one or more of folds 120, and to thereby radially enlarge lumen 118 of distal end portion 114 an amount sufficient to accommodate the passage of the distal end of surgical instrument "I" therethrough.

In an alternative method of use, instrument introducer 100 is initially placed on the distal end portion of surgical instrument "I". With instrument introducer 100 positioned thereon, the distal end portion of surgical instrument "I" is inserted and/or introduced into the cavity, orifice and/or body opening of the patient to the desired depth and/or position. With instrument introducer 100 held at the desired depth, surgical instrument "I" is distally advanced relative to instrument introducer 100 causing distal end portion 114 to radially expand and/or stretch. Distal end portion 114 radially expands an amount sufficient to accommodate the passage of the distal end of surgical instrument "I" therethrough.

In either method, it is understood by those skilled in the art that flange 122 will limit the depth of penetration of instrument introducer 100 into the body orifice of the patient. Additionally, in either method, it is preferred that a lubricant (e.g., petroleum jelly, water-based lubricants, glycerin, etc.), compatible with the materials of the introducer, surgical instrument and patient, is applied to the inner surface of instrument introducer 100 and/or the outer surface of surgical instrument "I" in order to facilitate insertion of surgical instrument "I" through instrument introducer 100. Additionally, it is contemplated that a suitable lubricant can be applied to the outer surface of instrument introducer 100 and/or into the cavity or body orifice of the patient in order to facilitate insertion of instrument introducer 100 and/or surgical instrument "I" into and through instrument introducer 100.

## Claims

1. An instrument introducer (100, 200, 300, 400, 500) for facilitating the introduction of a surgical instrument into a body opening of a patient, the instrument introducer comprising:
a body portion (112) defining a lumen (118) for passing a portion of the surgical instrument therethrough, the body portion including:
a distal end portion (114) including a distal orifice (113);
a proximal end portion (116) including a proximal orifice (115); and
at least one fold (120) formed in at least the distal end portion of the body portion, **characterised in that**
the at least one fold provides the distal end portion with a taper extending in a distal direction.

2. The instrument introducer according to claim 1, wherein the distal end portion has a first condition having a generally conical shape.

3. The instrument introducer according to claim 2, wherein in the first condition, the distal end portion has a generally frusto-conical shape.

4. The instrument introducer according to any one of the preceding claims, wherein the at least one fold is a plurality of folds extending radially about the distal end portion.

5. The instrument introducer according to claim 4, wherein the folds extend in a longitudinal direction.

6. The instrument introducer according to claim 4 or 5, wherein the distal end portion has a first condition wherein the folds are unexpanded to facilitate insertion of the distal end portion into a body orifice.

7. The instrument introducer according to claim 6, wherein the distal end portion has one or more subsequent conditions wherein the folds are at least partially extended to accommodate the passage of a surgical instrument therethrough.

8. The instrument introducer according to claim 7, wherein the distal end portion has a subsequent condition having an extended internal diameter substantially equal to an outer diameter of a surgical instrument.

9. The instrument introducer according to any one of claims 4 to 8, wherein the folds are integral with one another.

10. The instrument introducer according to any one of claims 4 to 9, wherein the folds overlap one another.

11. The instrument introducer according to any one of the preceding claims, wherein the body portion has an inner diameter of from about 30 mm (1.2 inches) to about 40 mm (1.6 inches).

12. The instrument introducer according to any one of the preceding claims, wherein the instrument introducer is fabricated from at least one of a polypropylene, a moldable plastic, a thermoformable plastic, a polymer, a urethane, a silicone, a natural rubber, a synthetic rubber, an elastomer, and an elastomeric material, and a latex material.

13. The instrument introducer according to any one of the preceding claims, wherein at least one fold (220) extends along the entire length of the body portion.

14. The instrument introducer according to any one of the preceding claims, wherein the fold is in the form of a pleat.

15. The instrument introducer according to any one of the preceding claims, wherein the body portion includes at least one annular fold (320) formed about the body portion.

16. The instrument introducer according to claim 5 or any one of claims 6 to 15 when dependent on claim 5, wherein the distal ends of the folds are radially longer than their proximal ends.

## Patentansprüche

1. Instrumenteninserter (100, 200, 300, 400, 500) zur Erleichterung des Einführens eines chirurgischen Instruments in eine Körperöffnung eines Patienten, wobei der Instrumenteninserter umfasst:
einen Körperabschnitt (112), der ein Lumen (118) für den Durchtritt eines Teils des chirurgischen Instruments dort durch definiert, wobei der Körperabschnitt aufweist:
einen flexiblen distalen Endabschnitt (114) mit einer distalen Öffnung (113);
einen proximalen Endabschnitt (116) mit einer proximalen Öffnung (115); und
mindestens eine Einknickung (120), die zumindest im distalen Endabschnitt des Körperabschnitts ausgebildet ist, **dadurch gekennzeichnet, dass**
die mindestens eine Einknickung den distalen Endabschnitt mit einer Verjüngung versieht, die sich in distaler Richtung zur distalen Öffnung (113) erstreckt.

2. Instrumenteninserter nach Anspruch 1, wobei der distale Endabschnitt einen ersten Zustand mit allgemein konischer Form besitzt.

3. Instrumenteninserter nach Anspruch 2, wobei der distale Endabschnitt im ersten Zustand allgemein kegelstumpfförmig ist.

4. Instrumenteninserter nach einem der vorangehenden Ansprüche, wobei die mindestens eine Einknickung eine Vielzahl von Einknickungen ist, die sich radial um den distalen Endabschnitt erstrecken.

5. Instrumenteninserter nach Anspruch 4, wobei sich die Einknickungen in einer longitudinalen Richtung erstrecken.

6. Instrumenteninserter nach Anspruch 4 oder 5, wobei der distale Endabschnitt einen ersten Zustand besitzt, in dem die Einknickungen nicht expandiert sind, um das Einführen des distalen Endabschnitts in eine Körperöffnung zu erleichtern.

7. Instrumenteninserter nach Anspruch 6, wobei der distale Endabschnitt einen oder mehrere nachfolgende Zustände besitzt, in denen die Einknickungen zumindest teilweise ausgedehnt sind, um dem Durchtritt eines chirurgischen Instruments dort durch Platz zu bieten.

8. Instrumenteninserter nach Anspruch 7, wobei der distale Endabschnitt einen nachfolgenden Zustand mit einem ausgedehnten Innendurchmesser besitzt, welcher im Wesentlichen gleich einem Außendurchmesser eines chirurgischen Instruments ist.

9. Instrumenteninserter nach einem der Ansprüche 4 bis 8, wobei die Einknickungen einstückig miteinander sind.

10. Instrumenteninserter nach einem der Ansprüche 4 bis 9, wobei die Einknickungen einander überlappen.

11. Instrumenteninserter nach einem der vorangehenden Ansprüche, wobei der Körperabschnitt einen Innendurchmesser von ungefähr 30 mm (1,2 Zoll) bis ungefähr 40 mm (1,6 Zoll) besitzt.

12. Instrumenteninserter nach einem der vorangehenden Ansprüche, wobei der Instrumenteninserter aus einem Polypropylen, einem formbaren Kunststoff, einem wärmeformbaren Kunststoff, einem Polymer, einem Urethan, einem Silikon, einem natürlichen Gummi, einem synthetischen Gummi, einem Elastomer, einem elastomerischen Material und/oder einem Latexmaterial hergestellt ist.

13. Instrumenteninserter nach einem der vorangehenden Ansprüche, wobei mindestens eine Einknickung (220) sich entlang der gesamten Länge des Körperabschnitts erstreckt.

14. Instrumenteninserter nach einem der vorangehenden Ansprüche, wobei die Einknickung in Form einer Falte ist.

15. Instrumenteninserter nach einem der vorangehenden Ansprüche, wobei der Körperabschnitt mindestens eine ringförmige Einknickung (320) umfasst, die um den Körperabschnitt herum ausgebildet ist.

16. Instrumenteninserter nach Anspruch 5, oder einem der vorangehenden Ansprüche 6 bis 15, wenn abhängig vom Anspruch 5, wobei die distalen Enden der Einknickungen radial länger sind als ihre proximalen Enden.

## Revendications

1. Module d'introduction d'instrument (100, 200, 300, 400, 500) pour faciliter l'introduction d'un instrument chirurgical dans une ouverture corporelle d'un patient, le module d'introduction d'instrument comprenant :
une portion de corps (112) définissant une lumière (118) pour faire passer une partie de l'instrument chirurgical à travers celle-ci, la portion de corps incluant : une portion d'extrémité distale flexible (114) incluant un orifice distal (113) ;
une portion d'extrémité proximale (116) incluant un orifice proximal (115) ; et
au moins un pli (120) formé dans au moins la portion d'extrémité distale de la portion de corps, **caractérisé en ce que**
au moins un pli précité réalise la portion d'extrémité distale avec une conicité s'étendant dans une direction distale vers ledit orifice distal (113).

2. Module d'introduction d'instrument selon la revendication 1, où la portion d'extrémité distale possède un premier état d'une forme généralement conique.

3. Module d'introduction d'instrument selon la revendication 2, où dans le premier état, la portion d'extrémité distale possède une forme généralement tronconique.

4. Module d'introduction d'instrument selon l'une des revendications précédentes, où au moins un pli précité est une pluralité de plis s'étendant radialement autour de la portion d'extrémité distale.

5. Module d'introduction d'instrument selon la revendication 4, où les plis s'étendent dans une direction longitudinale.

6. Module d'introduction d'instrument selon la revendication 4 ou 5, où la portion d'extrémité distale présente un premier état où les plis ne sont pas expansés pour faciliter l'insertion de la portion d'extrémité distale dans un orifice corporel.

7. Module d'introduction d'instrument selon la revendication 6, où la portion d'extrémité distale présente un ou plusieurs états suivants où les plis sont au moins partiellement étendus pour permettre le passage de l'instrument chirurgical à travers celle-ci.

8. Module d'introduction d'instrument selon la revendication 7, où la portion d'extrémité distale présente un état suivant ayant un diamètre interne étendu sensiblement égal à un diamètre extérieur d'un instrument chirurgical.

9. Module d'introduction d'instrument selon l'une des revendications 4 à 8, où les plis sont intégraux les uns avec les autres.

10. Module d'introduction d'instrument selon l'une des revendications 4 à 9, où les plis se chevauchent.

11. Module d'introduction d'instrument selon l'une des revendications précédentes, où la portion de corps présente un diamètre intérieur d'environ 30 mm (1,2 pouces) à environ 40 mm (1,6 pouces).

12. Module d'introduction d'instrument selon l'une des revendications précédentes, où le module d'introduction d'instrument est fabriqué à partir d'au moins un parmi un polypropylène, plastique moulable, plastique thermoformable, polymère, uréthanne, silicone, caoutchouc naturel, caoutchouc synthétique, élastomère et un matériau élastomère et un matériau latex.

13. Module d'introduction d'instrument selon l'une des revendications précédentes, où au moins un pli (220) s'étend sur toute la longueur de la portion de corps.

14. Module d'introduction d'instrument selon l'une des revendications précédentes, où le pli se présente sous la forme d'un pliage.

15. Module d'introduction d'instrument selon l'une des revendications précédentes, où la portion de corps comprend au moins un pli annulaire (320) formé autour de la portion de corps.

16. Module d'introduction d'instrument selon la revendication 5 ou l'une quelconque des revendications 6 à 15 lorsqu'elle dépend de la revendication 5, où les extrémités distales des plis sont radialement plus longues que leurs extrémités proximales.
